(19) Europäisches Patentamt
European Patent Office
Office européen des brevets

(11) Veröffentlichungsnummer: **0 281 909**
**A2**

(12) # EUROPÄISCHE PATENTANMELDUNG

(21) Anmeldenummer: **88103121.5**

(51) Int. Cl.⁴: **C07C 147/06**, A01N 47/24

(22) Anmeldetag: **02.03.88**

(30) Priorität: **11.03.87 DE 3707687**

(43) Veröffentlichungstag der Anmeldung:
**14.09.88 Patentblatt 88/37**

(84) Benannte Vertragsstaaten:
**BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Fest, Christa, Dr.**
**Im Johannistal 20**
**D-5600 Wuppertal 1(DE)**
Erfinder: **Brandes, Wilhelm, Dr.**
**Eichendorffstrasse 3**
**D-5653 Leichlingen(DE)**
Erfinder: **Dutzmann, Stefan, Dr.**
**Leinenweberstrasse 33**
**D-4000 Düsseldorf 13(DE)**
Erfinder: **Hänssler, Gerd, Dr.**
**Am Arenzberg 58a**
**D-5090 Leverkusen 3(DE)**
Erfinder: **Kuck, Karl-Heinz, Dr.**
**Heerstrasse 24**
**D-4018 Langenfeld(DE)**

(54) **Benzaldoxim-carbamat-Derivate.**

(57) Benzaldoxim-carbamat-Derivate der Formel (I)

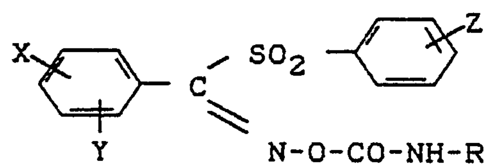

(I)

in welcher
X für Wasserstoff oder Halogen steht,
Y für Halogen oder Alkyl steht,
Z für Wasserstoff, Halogen, Alkyl, Alkoxy oder Halogenalkyl steht und
R für Alkyl, Halogenalkyl, Cyanalkyl, jeweils gegebenenfalls ein-bis mehrfach, gleich oder verschieden substituiertes Phenyl oder Phenylalkyl, Tosyl oder für gegebenenfalls ein-bis mehrfach, gleich oder verschieden substituiertes Cycloalkyl steht,
ausgenommen die Verbindungen, in denen X für 2-Chlor, Y für 6-Chlor, Z für 4-Methyl und R für die in der Beschreibung angegebenen Reste stehen, und ihre Verwendung zur Bekämpfung von Schädlingen, außerdem einige der Ausgangsverbindungen, die neu sind.

Die neuen Benzaldoxim-carbamat-Derivate der Formel (I) können nach bekannten Verfahren hergestellt werden, z. B. indem man geeignete α-Phenylsulfonyl-benzaldoxime mit geeigneten Isocyanaten umsetzt. Die

EP 0 281 909 A2

noch neuen Ausgangsverbindungen können z. B. aus geeigneten Halogenbenzaldoximen mit Phenylsulfinsäuren hergestellt werden.

## Benzaldoxim-carbamat-Derivate

Die vorliegende Erfindung betrifft neue Benzaldoxim-carbamat-Derivate, ein Verfahren zu ihrer Herstellung und ihre Verwendung als Schädlingsbekämpfungsmittel, außerdem zum Teil noch neue Vorprodukte.

Es sind bereits eine Reihe von Benzaldoximen, wie z.B. das α-Phenylsulfonyl-2,6-dichlor-benzaldoxim, bekannt. Ebenso wie deren Verwendung als Pflanzenschutzmittel, vor allem ist ihre Verwendung in Mitteln zur Bekämpfung von Weizensteinbrand (vgl. Schweizer Patent Nr. 423.350) bekannt.

Weiterhin sind Benzaldoxim-carbamat-Derivate als Fungizide bekannt (vgl. DE-OS 3 520 943).

Ihre Wirkung ist jedoch unter bestimmten Bedingungen nicht immer voll befriedigend, z.B. bei niedrigen Aufwandmengen.

Es wurden neue Benzaldoxim-carbamat-Derivate der Formel (I)

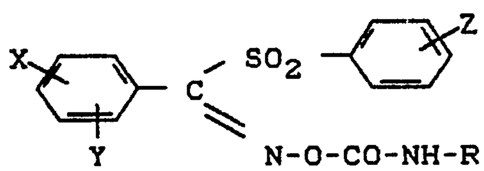

(I)

gefunden, in welcher

X für Wasserstoff oder Halogen steht,

Y für Halogen oder Alkyl steht,

Z für Wasserstoff, Halogen, Alkyl, Alkoxy oder Halogenalkyl steht und

R für Alkyl, Halogenalkyl, Cyanalkyl, jeweils gegebenenfalls ein-bis mehrfach, gleich oder verschieden substituiertes Phenyl oder Phenylalkyl, Tosyl oder für gegebenenfalls ein-bis mehrfach, gleich oder verschieden substituiertes Cycloalkyl steht,

ausgenommen die Verbindungen, in denen X für 2-Chlor, Y für 6-Chlor, Z für 4-Methyl und R für die oben angegebenen Reste stehen.

Weiterhin wurde gefunden, daß man die Benzaldoxim-carbamat-Derivate der Formel (I)

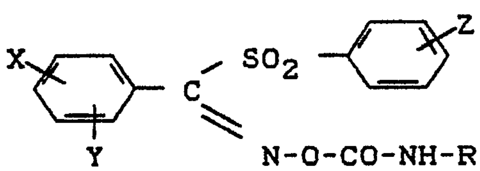

(I)

in welcher

X für Wasserstoff oder Halogen steht,

Y für Halogen oder Alkyl steht,

Z für Wasserstoff, Halogen, Alkyl, Alkoxy oder Halogenalkyl steht und

R für Alkyl, Halogenalkyl, Cyanalkyl, jeweils gegebenenfalls ein-bis mehrfach, gleich oder verschieden substituiertes Phenyl oder Phenylalkyl, Tosyl oder für gegebenenfalls ein-bis mehrfach, gleich oder verschieden substituiertes Cycloalkyl steht,

ausgenommen die Verbindungen, in denen X für 2-Chlor, Y für 6-Chlor, Z für 4-Methyl und R für die oben angegebenen Reste stehen,

erhält, wenn man α-Phenylsulfonly-benzaldoxime der Formel (II)

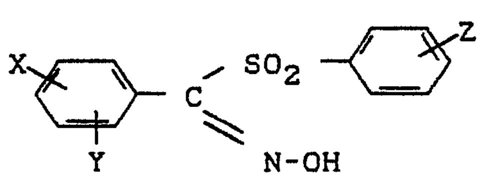

(II)

mit Isocyanaten der Formel (III)

R-NCO (III)

in welchen
X, Y, Z und R die oben angegebenen Bedeutungen haben,
gegebenenfalls in Gegenwart von Lösungs-oder Verdünnungsmitteln bei Temperaturen von 0°C bis 100°C umsetzt.

Die erfindungsgemäßen Benzaldoxim-carbamat-Derivate der Formel (I) weisen starke biologische, vor allem fungizide Eigenschaften auf.

Überraschenderweise zeigen dabei die erfindungsgemäßen Verbindungen eine erheblich höhere, vor allem fungizide Wirksamkeit als die aus dem Stand der Technik bekannten Verbindungen, die strukturell und/oder wirkungsmäßig sehr naheliegende Verbindungen sind.

Die erfindungsgemäßen Verbindungen der Formel (I) können als syn-oder anti-Isomere oder als deren Gemische in unterschiedlicher Zusammensetzung anfallen. Die Erfindung bezieht sich sowohl auf die reinen Isomeren als auch auf die Isomerengemische.

Die erfindungsgemäßen Benzaldoxim-carbamat-Derivate sind durch die Formel (I) allgemein definiert. Bevorzugt sind Verbindungen der Formel (I), in welcher
X für Wasserstoff oder Halogen steht,
Y für Halogen oder geradkettiges oder verzweigtes Alkyl mit 1 bis 4 Kohlenstoffatomen steht,
Z für Wasserstoff, Halogen, jeweils geradkettiges oder verzweigtes Alkyl, Alkoxy oder Halogenalkyl mit jeweils 1 bis 4 Kohlenstoffatomen und gegebenenfalls 1 bis 9 gleichen oder verschiedenen Halogenatomen steht und
R für geradkettiges oder verzweigtes Alkyl mit 1 bis 6 Kohlenstoffatomen, für geradkettiges oder verzweigtes Halogenalkyl mit 1 bis 10 Kohlenstoffatomen und 1 bis 9 gleichen oder verschiedenen Halogenatomen, für Cyanalkyl mit 1 bis 8 Kohlenstoffatomen im Alkylteil, für jeweils gegebenenfalls ein-bis fünffach, gleich oder verschieden durch Halogen, geradkettiges oder verzweigtes Alkyl mit 1 bis 6 Kohlenstoffatomen, geradkettiges oder verzweigtes Alkoxy mit 1 bis 4 Kohlenstoffatomen, geradkettiges oder verzweigtes Halogenalkyl oder Halogenalkoxy mit jeweils 1 bis 4 Kohlenstoffatomen und jeweils 1 bis 9 gleichen oder verschiedenen Halogenatomen substituiertes Phenyl oder Phenylalkyl mit 1 bis 4 Kohlenstoffatomen im geradkettigen oder verzweigten Alkylteil, für Tosyl oder für gegebenenfalls ein-bis fünffach, gleich oder verschieden durch geradkettiges oder verzweigtes Alkyl mit 1 bis 4 Kohlenstoffatomen substituiertes Cycloalkyl mit 5 bis 7 Kohlenstoffatomen steht,
ausgenommen die Verbindungen, in denen X für 2-Chlor, Y für 6-Chlor, Z für 4-Methyl und R für die oben angegebenen Reste stehen.

Besonders bevorzugt sind Verbindungen der Formel (I), in welcher
X für Wasserstoff, Fluor oder Chlor steht,
Y für Fluor, Chlor, Methyl, Ethyl, n-oder i-Propyl steht,
Z für Wasserstoff, Fluor, Chlor, geradkettiges oder verzweigtes Alkyl, Alkoxy oder Halogenalkyl mit jeweils 1 bis 3 Kohlenstoffatomen und 1 bis 5 gleichen oder verschiedenen Halogenatomen, wie Fluor und Chlor, steht und
R für geradkettiges oder verzweigtes Alkyl mit 1 bis 4 Kohlenstoffatomen, wie Methyl, Ethyl, n-Propyl, i-Propyl, n-Butyl, i-Butyl, s-Butyl und t-Butyl, für geradkettiges oder verzweigtes Halogenalkyl mit 1 bis 8 Kohlenstoffatomen und 1 bis 5 gleichen oder verschiedenen Halogenatomen, wie Fluor, Chlor, Brom und Iod, z. B. 2-Chlorethyl, 3-Chlor-n-propyl, 4-Chlor-n-butyl, 4-Chlor-n-pentyl und 6-Chlor-n-hexyl, für Cyanalkyl mit 1 bis 6 Kohlenstoffatomen im Alkylteil, z. B. 3-oder 5-Cyan-n-pentyl, für gegebenenfalls ein-bis dreifach, gleich oder verschieden durch Fluor, Chlor, geradkettiges oder verzweigtes Alkyl mit 1 bis 3 Kohlenstoffatomen, Alkoxy mit 1 oder 2 Kohlenstoffatomen, Halogenalkyl mit 1 oder 2 Kohlenstoffatomen und 1 bis 5 gleichen oder verschiedenen Halogenatomen und Halogenalkoxy mit 1 oder 2 Kohlenstoffatomen und 1 bis 5 gleichen oder verschiedenen Halogenatomen substituiertes Phenyl, für gegebenenfalls ein-bis dreifach durch Alkyl mit 1 bis 3 Kohlenstoffatomen substituiertes Phenylmethyl oder Phenylethyl, für Tosyl oder für gegebenenfalls ein-bis dreifach, gleich oder verschieden durch geradkettiges oder verzweigtes Alkyl mit 1 bis 3 Kohlenstoffatomen substituiertes Cycloalkyl mit 5 bis 7 Kohlenstoffatomen steht,
ausgenommen die Verbindungen, in denen X für 2-Chlor steht, Y für 6-Chlor steht, Z für 4-Methyl steht und R die oben angegebene Bedeutung hat.

Halogen steht für Fluor, Chlor, Brom oder Iod, vor allem für Fluor oder Chlor, wenn nicht anders angegeben. Ganz besonders bevorzugt sind Verbindungen der Formel (I), bei welchen
X für Wasserstoff, Fluor oder Chlor steht,
Y für Fluor, Chlor oder Methyl steht,
Z für Wasserstoff, Fluor, Chlor, Methyl, Methoxy, Trichlormethyl, Trifluormethyl oder Dichlorfluormethyl steht und
R für Methyl, Ethyl, n-Propyl, i-Propyl, n-Butyl, s-Butyl, i-Butyl, t-Butyl, 6-Chlor-n-hexyl, 5-Cyano-n-pentyl,

für ein-bis dreifach, gleich oder verschieden durch Methyl, Ethyl, n-Propyl, i-Propyl, Methoxy, Ethoxy, Trifluormethyl, Trifluormethoxy, Chlor und Fluor substituiertes Phenyl steht, für Benzyl, Tosyl, Cyclohexyl oder ein-bis dreifach, gleich oder verschieden durch Methyl oder Ethyl substituiertes Cyclohexyl steht, ausgenommen die Verbindungen, in denen X für 2-Chlor, Y für 6-Chlor, Z für 4-Methyl und R für die oben angegebenen Reste stehen.

Insbesondere seien Verbindungen der Formel (I) genannt, in welchen
X für 2-Chlor, 2-Fluor, 4-Chlor oder Wasserstoff steht,
Y für 4-Chlor, 6-Chlor, 6-Fluor oder 4-Methyl steht,
Z für Wasserstoff, 4-Chlor, 4-Fluor, 3-Trifluormethyl oder 4-Methoxy steht und
R für Methyl, Ethyl, n-Propyl, iso-Propyl, n-Butyl, iso-Butyl, tert.-Butyl, 6-Chlor-n-hexyl, 5-Cyano-n-pentyl, Phenyl, 4-Trifluormethoxy-phenyl, 2-, 3-oder 4-Methyl-phenyl, 3-Chlor-4-methyl-phenyl, 3-Methyl-4-chlorphenyl, 3-oder 4-Chlorphenyl, 3,4-oder 3,5-Dichlor-phenyl, 3-Trifluormethyl-phenyl, 4-Ethoxy-phenyl, 3-Chlor-4-trifluormethylphenyl oder 3,6-Di-isopropyl-phenyl, Benzyl, Tosyl, Cyclohexyl, 3,5,5-Trimethyl-cyclohexyl oder 4-Methyl-cyclohexyl steht und solche, bei welchen
X für 2-Fluor steht,
Y für 6-Fluor steht,
Z für Wasserstoff, 4-Chlor, 4-Fluor oder 4-Methyl steht und
R die oben angegebene Bedeutung hat.

Außerdem Verbindungen der Formel (I), in denen
X für Wasserstoff steht,
Y für 6-Chlor steht,
Z für 4-Methyl steht und
R für Methyl, Ethyl, n-Propyl, iso-Propyl, n-Butyl, iso-Butyl, tert.-Butyl, 6-Chlor-n-hexyl, 5-Cyano-n-pentyl, Phenyl, 4-Trifluormethoxy-phenyl, 2-, 3-oder 4-Methyl-phenyl, 3-Chlor-4-methyl-phenyl, 3-Methyl-4-chlorphenyl, 3-oder 4-Chlorphenyl, 3,4-oder 3,5-Dichlor-phenyl, 3-Trifluormethyl-phenyl, 4-Ethoxy-phenyl, 3-Chlor-4-trifluormethylphenyl oder 3,6-Di-isopropyl-phenyl, Benzyl, Tosyl, Cyclohexyl, 3,5,5-Trimethyl-cyclohexyl oder 4-Methyl-cyclohexyl steht.

Verwendet man α-(4-Methyl-phenylsulfonyl)-2-chlor-benzaldoxim und Methylisocyanat als Ausgangsstoffe, so läßt sich der Reaktionsablauf des erfindungsgemäßen Verfahrens durch das folgende Formelschema darstellen:

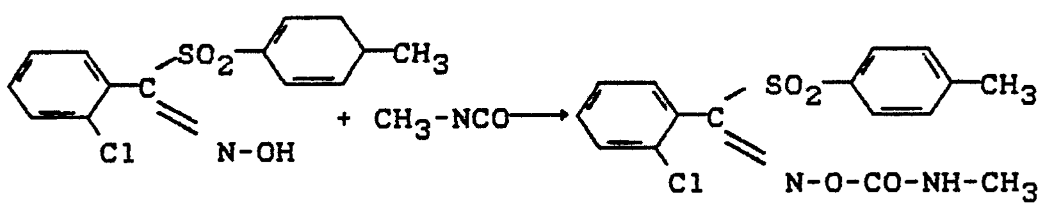

Die zur Durchführung des erfindungsgemäßen Verfahrens als Ausgangsstoffe benötigten α-Phenylsulfonyl-benzaldoxime sind durch die Formel (II) definiert. Diese Verbindungen sind teilweise bekannt, ebenso wie ihre Her stellung (vgl. z. B. US 3.234.255; CH 423.350; Synthesis 1974 (1), Seiten 49-51, Tetrahedron 1975, 31 (6), S. 597-600).

Neu und Teil der Erfindung sind die Verbindungen der Formel (IIA)

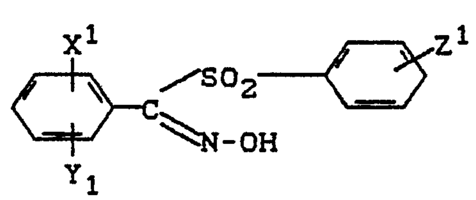

(IIA)

in welcher
$X^1$ für Wasserstoff, 2-Fluor oder 2-Chlor steht,
$Y^1$ für 4-Chlor, 6-Chlor oder 6-Fluor steht,
$Z^1$ für 4-Fluor, 4-Chlor, 3-Trifluormethyl oder 4-Methoxy steht und
$Z^1$ außerdem für Wasserstoff oder 4-Methyl steht, wenn wenigstens einer der Reste $X^1$, $Y^1$ für Fluor steht.

Die bekannten und auch die neuen Verbindungen der Formeln (II) und (IIA) können erhalten werden, indem man α-Halogenbenzaldoxime der Formel (IV) bzw. (IVA)

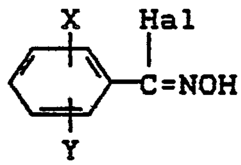

(IV)

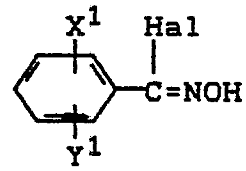

(IVA)

mit Phenylsulfinsäuren der Formeln (V) bzw. (VA)

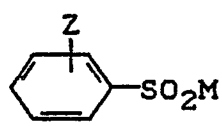

(V)

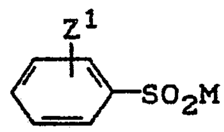

(VA)

in welchen
X, Y, Z, $X^1$, $Y^1$ und $Z^1$ die oben angegebenen Bedeutungen haben,
Hal für Halogen, vorzugsweise Chlor, steht und
M für Wasserstoff oder ein Alkalimetalläquivalent steht, gegebenenfalls in Gegenwart eines Lösungsmittels und gegebenenfalls in Gegenwart eines Säurebindemittels bei Temperaturen von 0°C bis 60°C umsetzt.

Auch die Verbindungen der Formeln (II) bzw. (IIA) zeigen bei entsprechenden Aufwandmengen eine fungizide Wirkung.

Das Verfahren zur Herstellung der Ausgangsverbindungen der Formeln (II) bzw. (IIA) kann gegebenenfalls in Gegenwart eines Lösungs-oder Verdünnungsmittels durchgeführt werden. Als solche kommen bevorzugt Alkohole, wie Methanol infrage.

Die Reaktionstemperaturen können in einem größeren Bereich variieren. Im allgemeinen arbeitet man zwischen 0 und 60°C, vorzugsweise zwischen 15 und 30°C.

Die weiterhin als Ausgangsstoffe benötigten Isocyanate sind durch die Formel (III) allgemein definiert. Es handelt sich dabei um bekannte Verbindungen der organischen Chemie.

Das erfindungsgemäße Verfahren kann gegebenenfalls in Gegenwart eines Lösungs-oder Verdünnungsmittels durchgeführt werden. Als solche kommen prinzipiell alle inerten organischen Lösungsmittel in Frage. Bevorzugt verwendet man Kohlenwasserstoffe, gegebenenfalls chlorierte, wie z.B. Benzol, Toluol, Xylol, Methylenchlorid, Chloroform, Tetrachlorkohlenstoff, Chlorbenzol und Dichlorbenzol.

Die Reaktionstemperaturen können in einem größeren Bereich variiert werden. Im allgemeinen arbeitet man zwischen 0 und 100°C, vorzugsweise zwischen 15 und 50°C.

Bei der Durchführung des erfindungsgemäßen Verfahrens läßt man die Reaktionspartner in etwa äquimolekularen Verhältnissen miteinander reagieren. Die Aufarbeitung des Reaktionsgemisches erfolgt in üblicher Weise, vornehmlich durch Absaugen, Waschen und Trocknen des ausgefallenen Reaktionsproduktes bzw. durch Einengen der Reaktionslösung und Verreiben des Rückstandes mit einem organischen Lösungsmittel, wie z. B. Ether.

Zum Ingangbringen der Reaktion können gegebenenfalls einige Tropfen einer Base, wie z.B. Triethylamin, zugegeben werden.

Die erfindungsgemäßen Wirkstoffe weisen eine starke biologische Wirkung auf und können zur Bekämpfung von unerwünschten Schädlingen praktisch eingesetzt werden. Die Wirkstoffe sind für den Gebrauch z. B. als Schädlingsbekämpfungsmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomondaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beipielsweise Xanthomonas campestris pv, oryzae;
Pseudomonas-Arten, wie beispielsweise Pseudomonas syringae pv. lachrymans;

Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea;
(Konidienform: Drechslera, Syn: Helminthosporium);
Cochliobolus-Arten, wie beispielsweise Cochliobolus sativus;
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Die erfindungsgemäßen Wirkstoffe lassen sich mit besonders gutem Erfolg zur Bekämpfung von Obst- und Gemüsekrankheiten, verursacht z. B. durch Phytophthora infestans, Venturia-Arten und Erwinia-Arten, zur Bekämpfung von Reiskrankheiten, verursacht durch Pyricularia oryzae und zur Bekämpfung von Getreidekrankheiten, verursacht z. B. durch Puccinia-Arten einsetzen.

Zu erwähnen sind auch die Wirkung gegen Getreidekrankheiten, verursacht z. B. durch Cochliobolus sativus, Pyrenophora teres und Leptophaeria nodonum, ebenso die gute bakterizide in-vitro-Wirkung und die Wirkung gegen Hygiene-und Vorratsschädlinge bei entsprechenden Anwendungskonzentrationen. Bei entsprechenden Konzentrationen und Anwendungen zeigen einige Verbindungen auch eine herbizide Wirkung.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungs mittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse

Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier-und/oder -schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Anwendungsbeispiele

In den folgenden Anwendungsbeispielen werden die nachstehend aufgeführten Verbindungen als Vergleichssubstanzen eingesetzt:

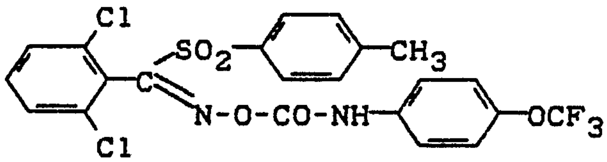 ;

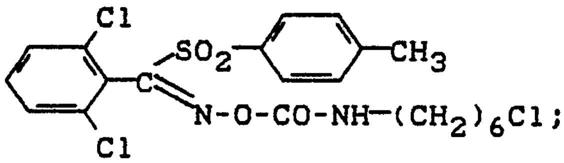

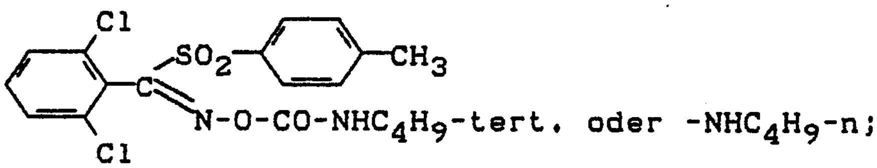

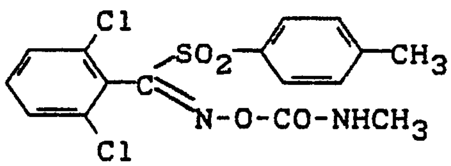 ;

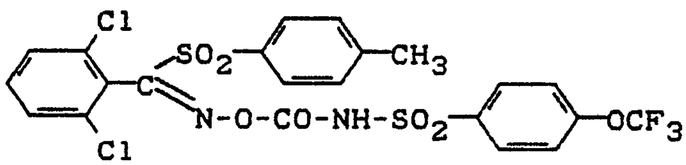 ;

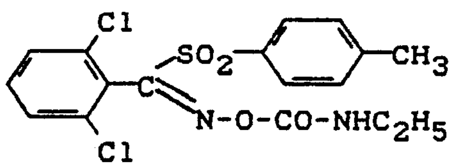 ;

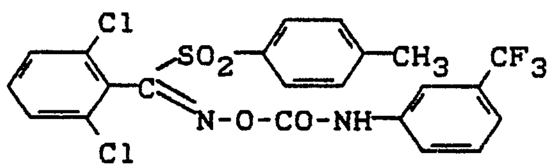 ;

(alle bekannt aus DE-OS 3 520 943).

Beispiel A

Pyricularia-Test (Reis)/protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auwertung des Krankheitsbefalls.

Die erfindungsgemäßen Verbindungen zeigen gegenüber dem Stand der Technik eine bessere Wirkung, so z. B. die Herstellungsbeispiele: 6, 14, 15, 16, 27, 20, 26, 28, 30, 31, 24, 19, 13 und 11.

Beispiel B

Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung.

Die Verbindungen sind denen aus dem Stand der Technik bekannten überlegen, z. B. gemäß den Herstellungsbeispielen: 4, 32, 20, 29, 31, 21, 23 und 24.

Beispiel C

Phytophthora-Test (Tomate) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z. B. die Verbindungen gemäß den Herstellungsbeispielen: 27, 20, 29 und 23.

Beispiel D

Puccinia-Test (Weizen) / protektiv /

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita mit in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

Die Pflanzen werden in einem Gewüchshaus bei einer Temperatur von ca. 20°C und einer relativen Luftfeuchtigkeit von ca. 80 % aufgestellt, um die Entwicklung von Rostpusteln zu begünstigen.

Beispiel E

Erwinia amylovora-Test / Bakteriose / Apfel / protektiv

Lösungsmittel: 49 Gewichtsteile Aceton
Emulgator: 1 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen durch Übersprühen mit einer wäßrigen Suspension von Erwinia amylovora inokuliert. Nach einer 48-stündigen Inkubation bei 100 % rel. Luftfeuchtigkeit verbleiben die Pflanzen 8 Tage bis zur Auswertung in einer Klimakammer bei 24°C und 70 bis 80 % rel. Luftfeuchtigkeit.

Eine deutliche Überlegenheit in der Wirksamkeit gegenüber dem Stand der Technik zeigen in diesem Test z.B. die Verbindungen gemäß den Herstellungsbeispielen: 26 und 23.

Herstellungsbeispiele

Beispiel 1

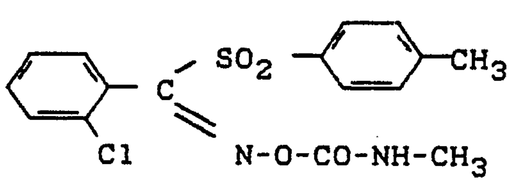

(I)

31 g (0,1 m) α-(4-Methyl-phenylsulfonyl)-2-chlorbenzaldoxim werden in 260 ml Methylenchlorid gelöst und bei Raumtemperatur mit 5,7 g (0,1 m) Methylisocyanat versetzt. Um die Reaktion in Gang zu bringen, werden einige Tropfen Triethylamin zugesetzt. Die Reaktion verläuft schwach exotherm. Man läßt das Reaktionsgemisch über Nacht bei Raumtemperatur rühren, es wird eine klare Lösung. Diese Lösung wird im Vakuum eingeengt, dabei bleibt ein amorpher Rückstand zurück. Er wird mit Ether verrieben und abgesaugt. Man erhält 19,8 g (54 % der Theorie) der gewünschten Substanz mit einem Schmelzpunkt von 166-169°C.

In analoger Weise wie Beispiel 1 werden die folgenden Verbindungen der Formel (I)

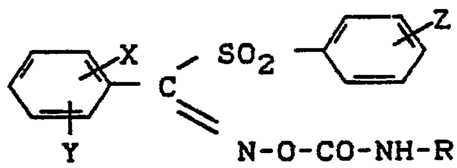

(I)

hergestellt:

| Bsp. | X 2-Stellung | Y 6-Stellung | Z 4-Stellung | R | Physikalische Daten (Schmelzpunkt °C) |
|---|---|---|---|---|---|
| 2 | Cl | Cl | H | 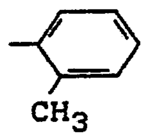 | 188 (Zers.) |
| 3 | Cl | Cl | H | 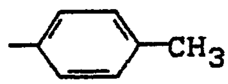 | 189 (Zers.) |
| 4 | Cl | Cl | H | 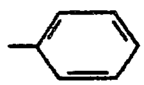 | 194 (Zers.) |
| 5 | Cl | Cl | F | $-C_2H_5$ | 182 |
| 6 | Cl | Cl | Cl | $-(CH_2)_6Cl$ | 118 |
| 7 | Cl | Cl | Cl | 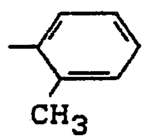 | 181 |
| 8 | Cl | Cl | Cl | 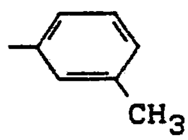 | 161 |
| 9 | Cl | Cl | Cl | 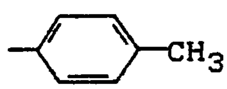 | 191 |
| 10 | F | F | H | 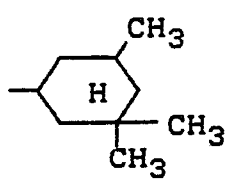 | 140 |
| 11 | F | F | H | $-C_2H_5$ | 143 |
| 12 | F | F | Cl | $-C_2H_5$ | 146 |

| Bsp. | X 2-Stellung | Y 6-Stellung | Z 4-Stellung | R | Physikalische Daten (Schmelzpunkt °C) |
|---|---|---|---|---|---|
| 13 | F | F | Cl | 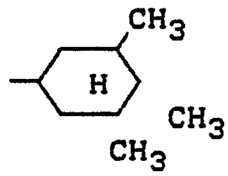 | 156 |
| 14 | Cl | Cl | H | $-C_2H_5$ | 184 |
| 15 | Cl | Cl | H | 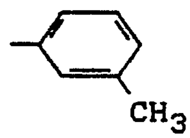 | 177 (Zers.) |
| 16 | Cl | Cl | H | 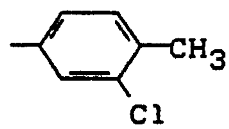 | 188 |
| 17 | Cl | Cl | H | 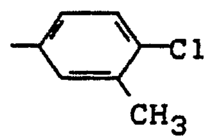 | 189 |
| 18 | Cl | F | $CH_3$ | 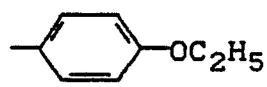 | 146 |
| 19 | F | F | $CH_3$ | $-C_2H_5$ | 153 |
| 20 | H | Cl | $CH_3$ | $-(CH_2)_6-Cl$ | 83 |
| 21 | H | Cl | $-CH_3$ | 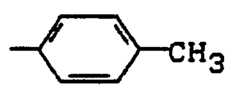 | 182 |
| 22 | H | Cl | $-CH_3$ | 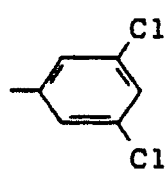 | 182 |

| Bsp. | X 2-Stellung | Y 6-Stellung | Z 4-Stellung | R | Physikalische Daten (Schmelzpunkt °C) |
|---|---|---|---|---|---|
| 23 | H | Cl | $-CH_3$ | $-C_4H_9$-n | 124 |
| 24 | H | Cl | $-CH_3$ | $-C_4H_9$-i | 140 |
| 25 | H | Cl | $-CH_3$ | 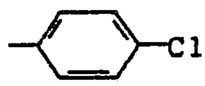 | 183 |
| 26 | H | Cl | $-CH_3$ | $-(CH_2)_5-CN$ | 83 |
| 27 | H | Cl | $-CH_3$ | $-C_2H_5$ | 146 |
| 28 | H | Cl | $-CH_3$ | 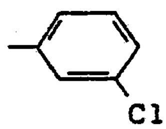 | 163 |
| 29 | H | Cl | $-CH_3$ | 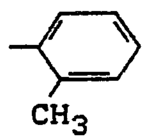 | 141 |
| 30 | H | Cl | $-CH_3$ | 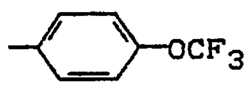 | 152 |
| 31 | H | Cl | $-CH_3$ | 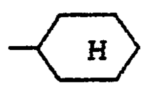 | 161 |
| 32 | H | Cl | $-CH_3$ | 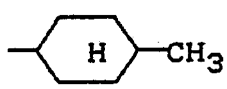 | 174 (Zers.) |
| 33 | Cl | Cl | Cl | $-C_3H_7$-i | 160 |
| 34 | H | Cl | $-CH_3$ | 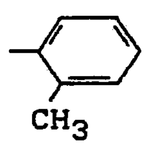 | 134 |

| Bsp. | X 2-Stellung | Y 6-Stellung | Z 4-Stellung | R | Physikalische Daten (Schmelzpunkt °C) |
|---|---|---|---|---|---|
| 35 | H | Cl | $-CH_3$ | 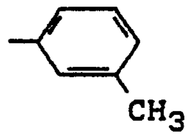 | 163 |
| 36 | Cl | Cl | H | 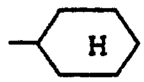 | 192 |
| 37 | Cl | Cl | H | 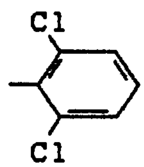 | 195 |
| 38 | Cl | Cl | H | $-CH_3$ | 175 |
| 39 | Cl | Cl | H | $-C_3H_7$-n | 168 |
| 40 | Cl | Cl | H | $-C_3H_7$-i | 170 (Zers.) |
| 41 | Cl | Cl | H | $-C_4H_9$-n | 158 |
| 42 | Cl | Cl | H | $-C_4H_9$-i | 166 |
| 43 | Cl | Cl | H | $-(CH_2)_5-CN$ | 132 |
| 44 | Cl | Cl | H | $-C_4H_9$-t | 162 (Zers.) |
| 45 | Cl | Cl | H | $-(CH_2)_6-Cl$ | 121 |
| 46 | Cl | Cl | H | $-CH_2-$ 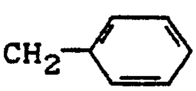 | 183 |
| 47 | Cl | Cl | H | 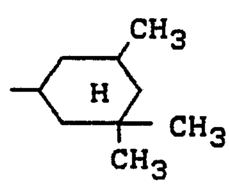 | 174 |

| Bsp. | X 2-Stellung | Y 6-Stellung | Z 4-Stellung | R | Physikalische Daten (Schmelzpunkt °C) |
|---|---|---|---|---|---|
| 48 | Cl | Cl | H | 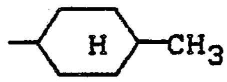 | 159 |
| 49 | Cl | Cl | H | 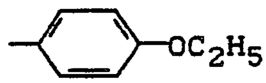 | 148 |
| 50 | Cl | Cl | H | 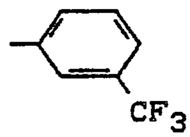 | 176 (Zers.) |
| 51 | Cl | Cl | H | 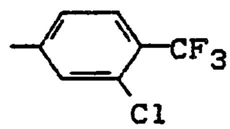 | 172 |
| 52 | Cl | Cl | H | 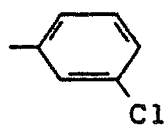 | 177 (Zers.) |
| 53 | Cl | Cl | H | 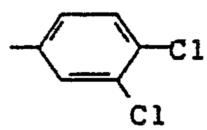 | 187 |
| 54 | Cl | Cl | H | 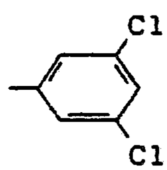 | 183 |
| 55 | Cl | Cl | H | 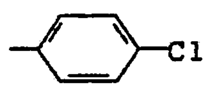 | 196 |
| 56 | Cl | F | $CH_3$ | 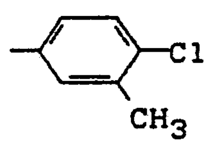 | |

| Bsp. | X 2-Stellung | Y 6-Stellung | Z 4-Stellung | R | Physikalische Daten (Schmelzpunkt °C) |
|---|---|---|---|---|---|
| 57 | Cl | Cl | Cl | 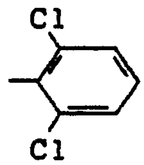 | 175 |
| 58 | Cl | Cl | Cl | 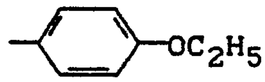 | 164 |
| 59 | Cl | Cl | Cl | 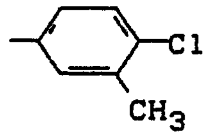 | 188 (Zers.) |
| 60 | Cl | Cl | Cl | 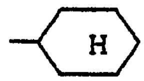 | 175 |
| 61 | Cl | Cl | Cl | 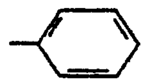 | 159 (Zers.) |
| 62 | Cl | Cl | Cl | $-CH_3$ | 185 |
| 63 | Cl | Cl | Cl | $-C_2H_5$ | 158 |
| 64 | Cl | Cl | Cl | $-C_3H_7$-n | 172 |
| 65 | Cl | Cl | Cl | $-C_4H_9$-n | 153 |
| 66 | Cl | Cl | Cl | $-C_4H_9$-i | 174 |
| 67 | Cl | Cl | Cl | $-C_4H_9$-t | 168 (Zers.) |
| 68 | Cl | Cl | Cl | $-(CH_2)_5-CN$ | 151 (Zers.) |
| 69 | Cl | Cl | Cl | $-CH_2$ 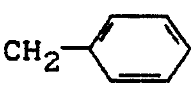 | 180 |

| Bsp. | X 2-Stellung | Y 6-Stellung | Z 4-Stellung | R | Physikalische Daten (Schmelzpunkt °C) |
|---|---|---|---|---|---|
| 70 | Cl | Cl | Cl | 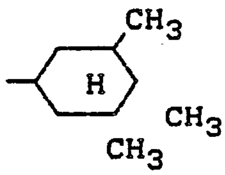 | 175 |
| 71 | Cl | Cl | Cl | 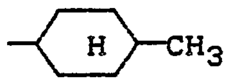 | 170 |
| 72 | Cl | Cl | Cl | 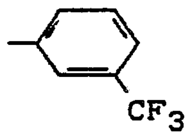 | 181 |
| 73 | Cl | Cl | Cl | 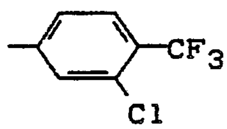 | 164 |
| 74 | Cl | Cl | F | 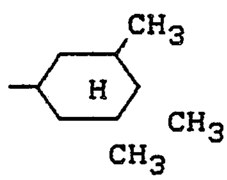 | 178 |
| 75 | H | Cl | $-CH_3$ | $-C_3H_7-n$ | 121 |
| 76 | Cl | Cl | $-OCH_3$ | 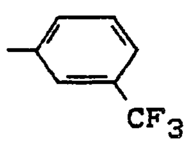 | 187 |
| 77 | F | F | $-CH_3$ | 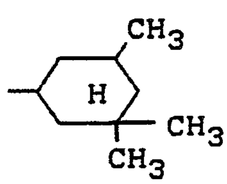 | 147 |
| 78 | H | Cl | $-CH_3$ | $-CH_2-$ 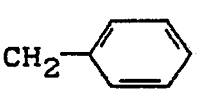 | 153 |

| Bsp. | X 2-Stellung | Y 6-Stellung | Z 4-Stellung | R | Physikalische Daten (Schmelzpunkt °C) |
|---|---|---|---|---|---|
| 79 | Cl | Cl | Cl | 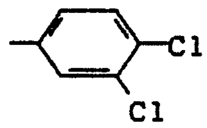 | 179 |
| 80 | H | Cl | $CH_3$ | 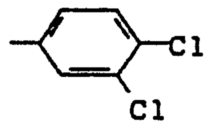 | 181 |
| 81 | Cl | Cl | Cl | 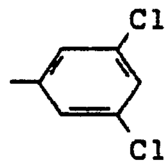 | 190 |
| 82 | F | F | $-CH_3$ | 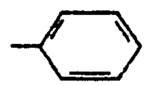 | 142 |
| 83 | H | Cl | $-CH_3$ | 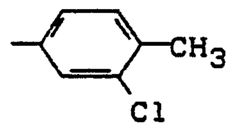 | 181 |
| 84 | H | Cl | $-CH_3$ | 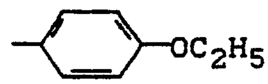 | 159 |
| 85 | H | Cl | $-CH_3$ | 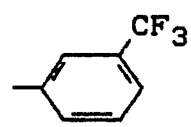 | 165 |
| 86 | Cl | Cl | H | Tosyl | 125 |
| 87 | H | Cl | $-CH_3$ | Tosyl | 65 |
| 88 | Cl | F | $-CH_3$ | $C_2H_5$ | 168 |
| 89 | Cl | F | $-CH_3$ | 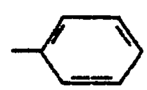 | 179 (Zers.) |

| Bsp. | X 2-Stellung | Y 6-Stellung | Z 4-Stellung | R | Physikalische Daten (Schmelzpunkt °C) |
|---|---|---|---|---|---|
| 90 | Cl | F | $-CH_3$ | 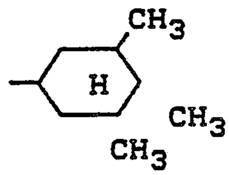 | 154 |
| 91 | Cl | F | $-CH_3$ | 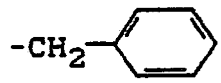 | 205 |
| 92 | Cl | F | $-CH_3$ | 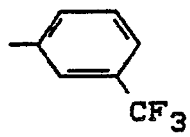 | 176 |
| 93 | Cl | F | $-CH_3$ | $-CH_3$ | 149 |
| 94 | Cl | F | $-CH_3$ | $-C_3H_7$-n | 157 |
| 95 | Cl | F | $-CH_3$ | $-C_3H_7$-i | 178 |
| 96 | Cl | F | $-CH_3$ | 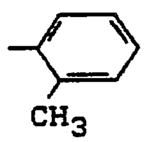 | 169 |
| 97 | Cl | F | $-CH_3$ | 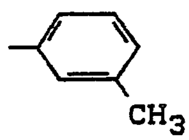 | 180 |
| 98 | Cl | F | $-CH_3$ | 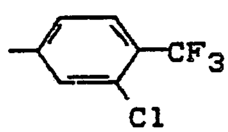 | |

| Bsp. | X 2-Stellung | Y 6-Stellung | Z 4-Stellung | R | Physikalische Daten (Schmelzpunkt °C) |
|---|---|---|---|---|---|
| 99 | Cl | F | $-CH_3$ | 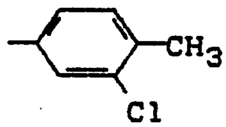 | 198 |
| 100 | F | F | $-CH_3$ | $-CH_3$ | 146 |
| 101 | F | F | $-CH_3$ | $-C_3H_7$-n | 132 |
| 102 | Cl | Cl | Cl | 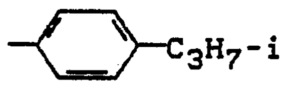 | 161 |
| 103 | H | Cl | H | $-CH_3$ | 98 |
| 104 | H | Cl | H | $-C_2H_5$ | 104 |
| 105 | H | Cl | H | $-C_3H_7$-n | 83 |
| 106 | H | Cl | H | $-C_3H_7$-i | 125 |
| 107 | H | Cl | H | $-C_4H_9$-i | 130 |
| 108 | H | Cl | H | $-C_4H_9$-sec. | 105 |
| 109 | H | Cl | Cl | $-CH_3$ | 125 |
| 110 | H | Cl | Cl | $-C_3H_7$-n | 75 |
| 111 | H | Cl | Cl | $-C_4H_9$-sec. | 104 |
| 112 | H | Cl | Cl | $-C_4H_9$-n | 109 |
| 113 | H | Cl | Cl | 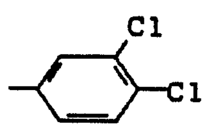 | 174 |
| 114 | H | Cl | Cl | 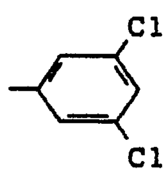 | 178 |

| Bsp. | X 2-Stellung | Y 6-Stellung | Z 4-Stellung | R | Physikalische Daten (Schmelzpunkt °C) |
|---|---|---|---|---|---|
| 115 | H | Cl | H | $-C_4H_9$-n | 102 |
| 116 | H | Cl | H | 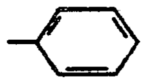 | 117 |
| 117 | Cl | F | Cl | $-CH_3$ | 131 |
| 118 | Cl | F | Cl | $-C_2H_5$ | 130 |
| 119 | Cl | F | Cl | $-C_3H_7$-n | 114 |
| 120 | Cl | F | Cl | $-C_3H_7$-i | 162 |
| 121 | Cl | F | Cl | $-C_4H_9$-n | 117 |
| 122 | Cl | F | Cl | $-C_4H_9$-sec. | 148 |
| 123 | H | Cl | H | 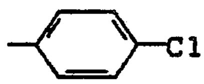 | 105 |
| 124 | H | Cl | H | 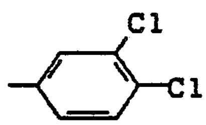 | 161 |
| 125 | H | Cl | H | 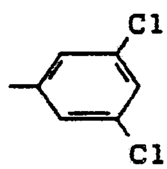 | 165 |
| 126 | H | Cl | H | 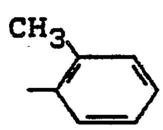 | 141 |
| 127 | H | Cl | H | 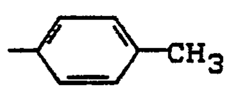 | 168 |
| 128 | Cl | F | Cl | $-C_4H_9$-i | 138 |
| 129 | Cl | F | Cl | $-(CH_2)_6Cl$ | 67 |

| Bsp. | X 2-Stellung | Y 6-Stellung | Z 4-Stellung | R | Physikalische Daten (Schmelzpunkt °C) |
|---|---|---|---|---|---|
| 130 | Cl | F | Cl | $-(CH_2)_5CN$ | 121 |
| 131 | H | Cl | Cl | 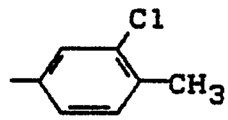 | 157 (Zers.) |
| 132 | H | Cl | Cl | 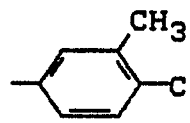 | 174 |
| 133 | H | Cl | Cl | 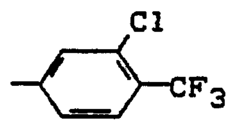 | 162 |
| 134 | H | Cl | Cl | 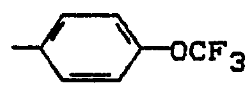 | 81 |
| 135 | H | Cl | Cl | 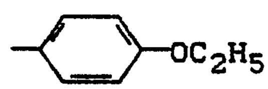 | 128 |
| 136 | H | Cl | Cl | 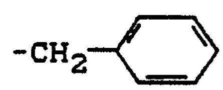 | 128 |
| 137 | H | Cl | H | 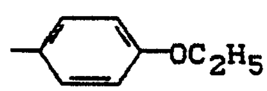 | 134 |
| 138 | H | Cl | H | 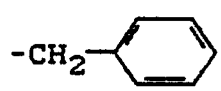 | 141 |
| 139 | H | Cl | H | 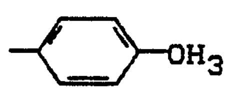 | 154 |
| 140 | H | Cl | Cl | 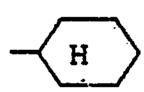 | 134 |

Herstellungsbeispiel für Verbindungen der Formel (II) bzw. (IIA)

Beispiel 1A

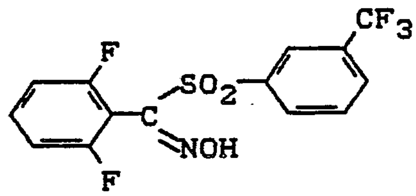

19,2 g (0,1 m) α-Chlor-2,6-difluor-benzaldoxim werden in 200 ml Methanol gelöst und mit 23,2 g (0,1 m) 3-Trifluormethylbenzolsulfinsaurem Natriumsalz bei Zimmertemperatur versetzt. Das Reaktionsgemisch wird ca. 15 Stunden bei dieser Temperatur gehalten, anschließend auf 1 l Eiswasser gegossen, ausgerührt, abgesaugt, gewaschen und getrocknet. Das Reaktionsprodukt wird aus Toluol umkristallisiert. Man erhält 24 g (62,5 % der Theorie) α-3-Trifluormethylphenylsulfonyl-2,6-difluorbenzaldoxim vom Schmelzpunkt 127°C.

Analog können hergestellt werden Verbindungen der Formel (II):

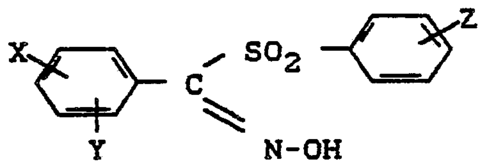

(II)

| Bsp. | X | Y | Z | Physikalische Daten (Schmelzpunkt °C) |
|---|---|---|---|---|
| 2 A | 4-Cl | 6-Cl | $4-CH_3$ | 143 |
| 3 A | 2-Cl | 6-F | 4-Cl | 158 |
| 4 A | 2-Cl | 6-F | $4-CH_3$ | 170 |
| 5 A | 2-Cl | 6-F | H | 143 |
| 6 A | 2-Cl | 6-F | $4-OCH_3$ | 145 |
| 7 A | 6-H | 2-Cl | $4-CH_3$ | 147 |
| 8 A | 2-Cl | 6-Cl | 4-H | 148 |
| 9 A | 2-Cl | 6-Cl | 4-F | 154 |
| 10 A | 2-Cl | 6-Cl | 4-Cl | 111 |
| 11 A | 2-F | 6-F | 4-H | 149 |
| 12 A | 2-F | 6-F | 4-Cl | 171 |
| 13 A | 2-F | 6-F | $4-CH_3$ | 166 |
| 14 A | 2-Cl | 6-Cl | $4-OCH_3$ | 163 |
| 15 A | 2-F | 6-F | $4-OCH_3$ | 145 |
| 16 A | 6-H | 2-Cl | $4-OCH_3$ | 141 |
| 17 A | 2-Cl | 6-Cl | $3-CF_3$ | 148 |
| 18 A | 2-F | 6-F | 4-F | 148 |
| 19 A | H | 4-Cl | $4-CH_3$ | 145 |
| 20 A | H | 2-Cl | H | 141 |
| 21 A | H | 2-Cl | 4-Cl | 150 |
| 22 A | 2-Cl | 6-F | 4-Cl | 158 |

## Ansprüche

1. Benzaldoxim-carbamat-Derivate der Formel (I)

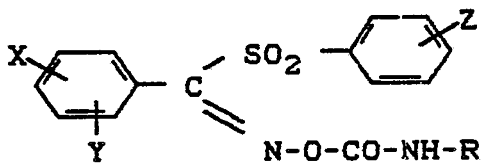

(I)

in welcher
X für Wasserstoff oder Halogen steht,
Y für Halogen oder Alkyl steht,
Z für Wasserstoff, Halogen, Alkyl, Alkoxy oder Halogenalkyl steht und
R für Alkyl, Halogenalkyl, Cyanalkyl, jeweils gegebenenfalls ein-bis mehrfach, gleich oder verschieden substituiertes Phenyl oder Phenylalkyl, Tosyl oder für gegebenenfalls ein-bis mehrfach, gleich oder verschieden substituiertes Cycloalkyl steht,
ausgenommen die Verbindungen, in denen X für 2-Chlor, Y für 6-Chlor, Z für 4-Methyl und R für die oben angegebenen Reste stehen.

2. Benzaldoxim-carbamat-Derivate gemäß Anspruch 1, worin in der Formel (I)
X für Wasserstoff oder Halogen steht,
Y für Halogen oder geradkettiges oder verzweigtes Alkyl mit 1 bis 4 Kohlenstoffatomen steht,
Z für Wasserstoff, Halogen, jeweils geradkettiges oder verzweigtes Alkyl, Alkoxy oder Halogenalkyl mit jeweils 1 bis 4 Kohlenstoffatomen und gegebenenfalls 1 bis 9 gleichen oder verschiedenen Halogenatomen steht und
R für geradkettiges oder verzweigtes Alkyl mit 1 bis 6 Kohlenstoffatomen, für geradkettiges oder verzweigtes Halogenalkyl mit 1 bis 10 Kohlenstoffatomen und 1 bis 9 gleichen oder verschiedenen Halogenatomen, für Cyanalkyl mit 1 bis 8 Kohlenstoffatomen im Alkylteil, für jeweils gegebenenfalls ein-bis fünffach, gleich oder verschieden durch Halogen, geradkettiges oder verzweigtes Alkyl mit 1 bis 6 Kohlenstoffatomen, geradkettiges oder verzweigtes Alkoxy mit 1 bis 4 Kohlenstoffatomen, geradkettiges oder verzweigtes Halogenalkyl oder Halogenalkoxy mit jeweils 1 bis 4 Kohlenstoffatomen und jeweils 1 bis 9 gleichen oder verschiedenen Halogenatomen substituiertes Phenyl oder Phenylalkyl mit 1 bis 4 Kohlenstoffatomen im geradkettigen oder verzweigten Alkylteil, für Tosyl oder für gegebenenfalls ein-bis fünffach, gleich oder verschieden durch geradkettiges oder verzweigtes Alkyl mit 1 bis 4 Kohlenstoffatomen substituiertes Cycloalkyl mit 5 bis 5 Kohlenstoffatomen steht,
ausgenommen die Verbindungen, in denen X für 2-Chlor, Y für 6-Chlor, Z für 4-Methyl und R für die oben angegebenen Reste stehen.

3. Benzaldoxim-carbamat-Derivate gemäß Anspruch 1, worin in der Formel (I)
X für Wasserstoff, Fluor oder Chlor steht,
Y für Fluor, Chlor, Methyl, Ethyl, n-oder i-Propyl steht,
Z für Wasserstoff, Fluor, Chlor, geradkettiges oder verzweigtes Alkyl, Alkoxy oder Halogenalkyl mit jeweils 1 bis 3 Kohlenstoffatomen und 1 bis 5 gleichen oder verschiedenen Halogenatomen steht und
R für geradkettiges oder verzweigtes Alkyl mit 1 bis 4 Kohlenstoffatomen für geradkettiges oder verzweigtes Halogenalkyl mit 1 bis 8 Kohlenstoffatomen und 1 bis 5 gleichen oder verschiedenen Halogenatomen für Cyanalkyl mit 1 bis 6 Kohlenstoffatomen im Alkylteil, für gegebenenfalls ein-bis dreifach, gleich oder verschieden durch Fluor, Chlor, geradkettiges oder verzweigtes Alkyl mit 1 bis 3 Kohlen stoffatomen, Alkoxy mit 1 oder 2 Kohlenstoffatomen, Halogenalkyl mit 1 oder 2 Kohlenstoffatomen und 1 bis 5 gleichen oder verschiedenen Halogenatomen und Halogenalkoxy mit 1 oder 2 Kohlenstoffatomen und 1 bis 5 gleichen oder verschiedenen Halogenatomen substituiertes Phenyl, für gegebenenfalls ein-bis dreifach durch Alkyl mit 1 bis 3 Kohlenstoffatomen substituiertes Phenylmethyl oder Phenylethyl, für Tosyl oder für gegebenenfalls ein-bis dreifach, gleich oder verschieden durch geradkettiges oder verzweigtes Alkyl mit 1 bis 3 Kohlenstoffatomen substituiertes Cycloalkyl mit 5 bis 7 Kohlenstoffatomen steht, ausgenommen die Verbindungen, in denen X für 2-Chlor steht, Y für 6-Chlor steht, Z für 4-Methyl steht und R die oben angegebene Bedeutung hat.

4. Benzaldoxim-carbamat-Derivate gemäß Anspruch 1, wobei in der Formel (I)
X für Wasserstoff, Fluor oder Chlor steht,
Y für Fluor, Chlor oder Methyl steht,

Z für Wasserstoff, Fluor, Chlor, Methyl, Methoxy, Trichlormethyl, Trifluormethyl oder Dichlorfluormethyl steht und

R für Methyl, Ethyl, n-Propyl, i-Propyl, n-Butyl, s-Butyl, i-Butyl, t-Butyl, 6-Chlor-n-hexyl, 5-Cyano-n-pentyl, für ein-bis dreifach, gleich oder verschieden durch Methyl, Ethyl, n-Propyl, i-Propyl, Methoxy, Ethoxy, Trifluormethyl, Trifluormethoxy, Chlor und Fluor substituiertes Phenyl steht, für Benzyl, Tosyl, Cyclohexyl oder ein-bis dreifach, gleich oder verschieden durch Methyl oder Ethyl substituiertes Cyclohexyl steht,

ausgenommen die Verbindungen, in denen X für 2-Chlor, Y für 6-Chlor, Z für 4-Methyl und R für die oben angegebenen Reste stehen.

5. Benzaldoxim-carbamat-Derivate gemäße Anspruch 1, worin in der Formel (I)

X für 2-Chlor, 2-Fluor, 4-Chlor oder Wasserstoff steht,

Y für 4-Chlor, 6-Chlor, 6-Fluor oder 4-Methyl steht,

Z für Wasserstoff, 4-Chlor, 4-Fluor, 3-Trifluormethyl oder 4-Methoxy steht und

R für Methyl, Ethyl, n-Propyl, iso-Propyl, n-Butyl, iso-Butyl, tert.-Butyl, 6-Chlor-n-hexyl, 5-Cyano-n-pentyl, Phenyl, 4-Trifluormethoxyphenyl, 2-, 3-oder 4-Methyl-phenyl, 3-Chlor-4-methyl-phenyl, 3-Methyl-4-chlorphenyl, 3-oder 4-Chlorphenyl, 3,4-oder 3,5-Dichlor-phenyl, 3-Trifluormethyl-phenyl, 4-Ethoxy-phenyl, 3-Chlor-4-trifluormethylphenyl oder 3,6-Diisopropyl-phenyl, Benzyl, Tosyl, Cyclohexyl, 3,5,5-Trimethyl-cyclohexyl oder 4-Methylcyclohexyl steht.

6. Verfahren zur Herstellung von Benzaldoxim-carbamat-Derivaten der Formel (I)

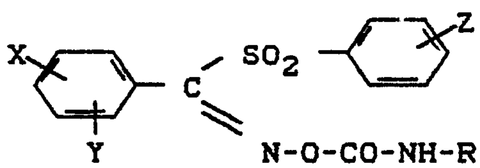

(I)

in welcher

X für Wasserstoff oder Halogen steht,

Y für Halogen oder Alkyl steht,

Z für Wasserstoff, Halogen, Alkyl, Alkoxy oder Halogenalkyl steht und

R für Alkyl, Halogenalkyl, Cyanalkyl, jeweils gegebenenfalls ein-bis mehrfach, gleich oder verschieden substituiertes Phenyl oder Phenylalkyl, Tosyl oder für gegebenenfalls ein-bis mehrfach, gleich oder verschieden substituiertes Cycloalkyl steht,

ausgenommen die Verbindungen, in denen X für 2-Chlor, Y für 6-Chlor, Z für 4-Methyl und R für die oben angegebenen Reste stehen, dadurch gekennzeichnet, daß man α-Phenyl-sulfonyl-benzaldoxime der Formel (II)

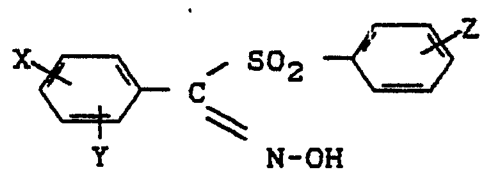

(II)

mit Isocyanaten der Formel (III)

R-NCO (III)

in welchen

X, Y, Z und R die oben angegebenen Bedeutungen haben,

gegebenenfalls in Gegenwart von Lösungs-oder Verdünnungsmitteln bei Temperaturen von 0°C bis 100°C umsetzt.

7. Schädlingsbekämpfungsmittel, gekennzeichnet durch einen Gehalt an mindestens einem Benzaldoxim-carbamat-Derivat der Formel (I) nach den Ansprüchen 1 und 6.

8. Verwendung von Benzaldoxim-carbamat-Derivaten der Formel (I) nach den Ansprüchen 1 und 6 zur Bekämpfung von Schädlingen.

9. Verfahren zur Bekämpfung von Schädlingen, dadurch gekennzeichnet, daß man Benzaldoxim-carbamat-Derivate der Formel (I) nach den Ansprüchen 1 und 6 auf Schädlinge und/oder ihren Lebensraum einwirken läßt.

10. Verfahren zur Herstellung von Schädlingsbekämpfungsmitteln, dadurch gekennzeichnet, daß man Benzaldoxim-carbamat-Derivate der Formel (I) nach den Ansprüchen 1 und 6 mit Streckmitteln und/oder oberflächenaktiven Mitteln vermischt.

11. Benzaldoxime der Formel (IIA)

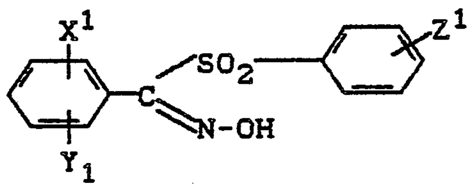

(IIA)

in welcher
$X^1$ für Wasserstoff, 2-Fluor oder 2-Chlor steht,
$Y^1$ für 4-Chlor, 6-Chlor oder 6-Fluor steht,
$Z^1$ für 4-Fluor, 4-Chlor, 3-Trifluormethyl oder 4-Methoxy steht und
$Z^1$ außerdem für Wasserstoff oder 4-Methyl steht, wenn wenigstens einer der Reste $X^1$, $Y^1$ für Fluor steht.

12. Verfahren zur Herstellung von Benzaldoximen der Formel (IIA)

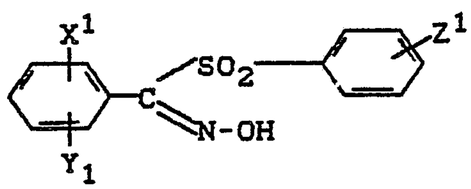

(IIA)

in welcher
$X^1$ für Wasserstoff, 2-Fluor oder 2-Chlor steht,
$Y^1$ für 4-Chlor, 6-Chlor oder 6-Fluor steht,
$Z^1$ für 4-Fluor, 4-Chlor, 3-Trifluormethyl oder 4-Methoxy steht und
$Z^1$ außerdem für Wasserstoff oder 4-Methyl steht, wenn wenigstens einer der Reste $X^1$, $Y^1$ für Fluor steht, dadurch gekennzeichnet, daß man α-Halogenbenzaldoxime der Formel (IVA)

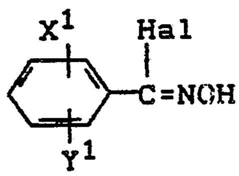

(IVA)

mit Phenylsulfinsäuren der Formel (VA)

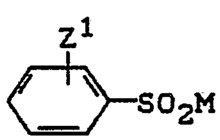

(VA)

in welchen
$X^1$, $Y^1$ und $Z^1$ die oben angegebenen Bedeutungen haben,
Hal für Halogen steht und
M für Wasserstoff oder ein Alkalimetalläquivalent steht, gegebenenfalls in Gegenwart eines Lösungsmittels und gegebenenfalls in Gegenwart eines Säurebindemittels bei Temperaturen von 0°C bis 60°C umsetzt.

13. Verwendung von Benzaldoximen der Formel (IIA) gemäß den Ansprüchen 11 und 12 zur Bekämpfung von Schädlingen.